(19) Europäisches Patentamt
European Patent Office
Office européen des brevets

(11) Publication number: **0 517 225 A1**

# (12) EUROPEAN PATENT APPLICATION

(21) Application number: **92109480.1**

(51) Int. Cl.[5]: **C07F 15/00**, A61K 31/28

(22) Date of filing: **04.06.92**

(30) Priority: **05.06.91 ES 9101355**
**14.05.92 ES 9201002**

(43) Date of publication of application:
**09.12.92 Bulletin 92/50**

(84) Designated Contracting States:
**BE CH DE FR GB GR IT LI NL PT**

(71) Applicant: **FERRER INTERNACIONAL, S.A.**
**Gran Via Carlos III, 94**
**08028 Barcelona(ES)**

(72) Inventor: **Foguet, Rafael**
**Llusanes 8**
**E-08022 Barcelona(ES)**
Inventor: **Sampedro, Federico**
**Polonia 2-4**
**E-08024 Barcelona(ES)**
Inventor: **Molins, Antonio M.**
**Caritat 5, Manresa**
**Barcelona(ES)**
Inventor: **Ruiz, José I.**
**Av. Gaudi, 51**
**E-08025 Barcelona(ES)**
Inventor: **Santal , Pilar**
**Polonia 2-4**
**E-08024 Barcelona(ES)**
Inventor: **Bonal, Joaquin**
**Reus 26**
**E-08022 Barcelona(ES)**
Inventor: **Ortiz, José A.**
**Corçega 429**
**E-08037 Barcelona(ES)**
Inventor: **Castell , Josep M.**
**Princep d'Astùries, 35**
**E-08012 Barcelona(ES)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

## (54) Aminopyrrolidine platinum compounds, a process for preparing them and pharmaceutical compositions containing them.

(57) The present invention provides compounds of the formula

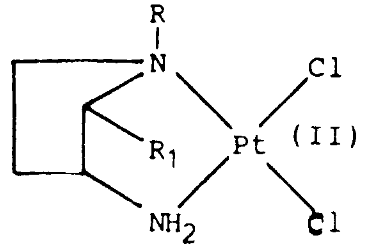

(I)

by treatment of a potassium tetrachloroplatinite solution in water with the corresponding aminopyrrolidine. In the compounds of the present invention R is a linear alkyl group containing 1 to 12 carbon atoms and $R_1$ is a linear alkyl group containing 1 to 3 carbon atoms. The compounds of the present invention have pharmacological application due to their antitumor activity.

EP 0 517 225 A1

The present invention relates to aminopyrrolidine platinum compounds of the general formula (I):

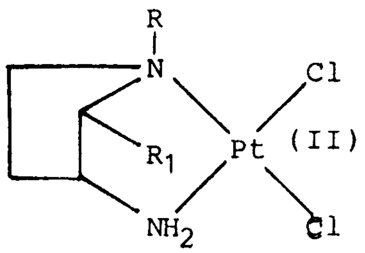

(I)

wherein R is a linear or branched alkyl group containing 1 to 12 carbon atoms and $R_1$ is a linear or branched alkyl group containing 1 to 3 carbon atoms, a process for preparing them and pharmaceutical compositions containing them.

The compounds of the present invention are prepared in accordance with the following reaction scheme:

$K_2PtCl_4$ +

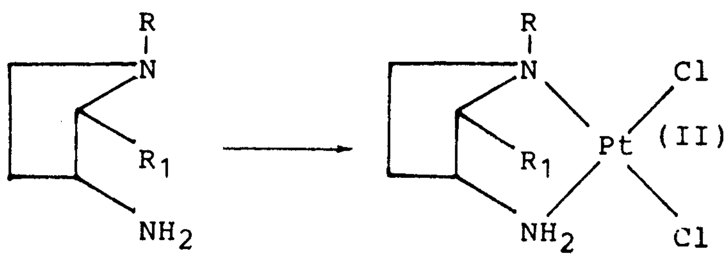

+ 2 KCl

(II) (I)

by treating potassium tetrachloroplatinite ($K_2PtCl_4$) in water with the corresponding aminopyrrolidine (II) (preferably in 1:1 stoichiometric proportion). The reaction is preferably carried out for about 16 to about 30 hours, in particular for about 24 hours, at room temperature and under inert atmosphere in order to avoid degradation of the free amine by oxidation or carbonation.

In turn, the starting aminopyrrolidines of the general formula (II) are obtained with respective 2-substituted pyrroles (III) according to the following reactions:

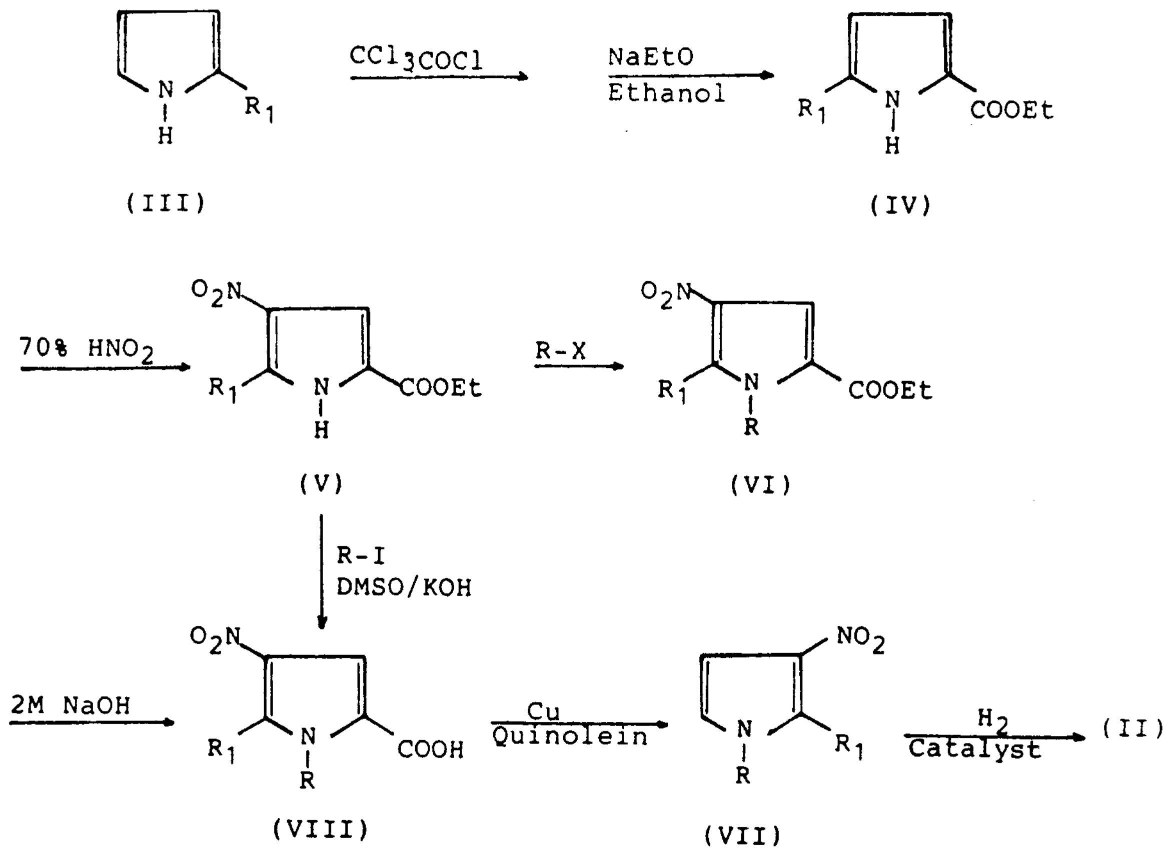


(III) (IV) (V) (VI) (VIII) (VII) (II)

According to the above scheme, the ethoxycarbonyl group in (IV) acts as a blocking protector of the 2-position of the pyrrole ring and as a directing group in the subsequent 4-position nitration reaction. The pyrrole ring N-alkylation in molecule (IV) with the corresponding halide occurs under phase-transfer catalysis conditions. Ethyl 5-alkyl-4-nitro-2-pyrrol-carboxylates (V) may be alkylated through their potassium salt with alkyl halides (R-X), wherein X is a halogen selected preferably from either bromine or iodine, by using hexadecyltributylphosphonium as a catalyst. Alternately, the alkylation of (V) with respective alkyl iodides in alkaline medium progresses towards the corresponding 1,5-dialkyl-4-nitro-2-pyrrolcarboxylic acids (VIII); these latter can also be reached by conventional saponification from (VI). Decarboxylation of (VIII) with metallic copper and quinolein leads to respective 1,2-dialkyl-3-nitropyrroles of the general formula (VII). Finally, the catalytic hydrogenation of (VII) under pressure provides the starting aminopyrrolidines of the general formula (II). These aminopyrrolidines, which are used as ligand in the reaction with potassium tetrachloroplatinite, are usually oils and hardly crystallize as such. However, they can react with picric acid to afford respective crystalline dipicrates which becomes a useful process for their characterization.

Applicants have found out that the compounds of the present invention have antitumor activity which has been demonstrated in mutagenicity tests. A summary of results in mutagenicity and acute toxicity tests is given hereinbelow.

a) Mutagenicity tests: E.coli bacterial strain, AB2463 mutant strain (RecA) was used. Mortality of this strain treated with compounds of the present invention was found to be similar to that of cisplatin. RecA strain, AB2463 mutant strain, which is defective in the recombination and induction of SOS system, was treated for 2 hours at 37ºC with compounds of the present invention. They caused similar mortality to that of carboplatin at significantly lower doses (Table 1).

TABLE 1

| Compounds | $LD_{10}$($\mu$M) | $LD_{50}$($\mu$M) | $LD_{90}$($\mu$M) |
|---|---|---|---|
| Cisplatin | 5.72 | 17.0 | 56.0 |
| Example 1 | >1.2 | 5.98 | 13.15 |
| Example 2 | 15.86 | 57.08 | 301.27 |
| Example 3 | 10.74 | 17.89 | 35.78 |
| Example 4 | 18.97 | 35.57 | 72.33 |
| Carboplatin | 71.53 | 606.0 | 1599 |

These results indicate that the activity of compounds of the present invention is similar as and superior to that of cisplatin and carboplatin.

b) Acute toxicity tests: $LD_{50}$ i.p. was determined in 6-7 week-old male $BDF_1$ mice, weighing 18-22 g, by using the method by Litchfield-Wilcoxon (J.Pharmacol.Ther., 96, 99-113, 1949), Results are shown in Table 2.

TABLE 2

| Compound | $LD_{50}$(mg/kg) |
|---|---|
| Example 1 | 95.02 |
| Example 2 | 60.95 |
| Example 3 | 82.72 |
| Example 4 | 113.81 |
| Cisplatin | 17.5 |
| Carboplatin | 117.5 |

Taking into account that the contribution of carboplatin to therapy was subsequent to cisplatin and has represented a therapeutic advantage versus the latter due to its low toxicity (although it is less active), the compounds of the present invention also afford a therapeutic advantage versus carboplatin because they are more active and keep a similar toxicity range.

In conclusion, the compounds of the presente invention are useful in the treatment of different malignant tumors, like lung neoplasms, stomach neoplasms, mammary neoplasms, testicular neoplasms, ovarian neoplasms, bladder neoplasms, brain and neck neoplasms, osteosarcoma, etc. These compounds may be administered,mixed with suitable carriers, at daily doses ranging from 20 to 100 mg/kg.

A number of examples will now be described in non-limitative manner to illustrate the invention.

**EXAMPLE 1:**

Cis-dichloro(3-amino-1,2-dimethylpyrrolidine)platinum$^{(II)}$ (I, R = $R_1$ = Me)

A 0.1M solution of ligand (1 equivalent) was added to an aqueous solution subjected to $K_2PtCl_4$ stirring (1 equivalent). The system was kept continously under argon atmosphere and distilled water was added until a concentration of 0.05 M 3-amino-1,2-dimethylpyrrolidine was obtained. The solution was acidified to pH 1.2 by adding 2M HCl. The residue was concentrated to dryness and crystallized with 0.1M HCl to give a yellow solid (Yield 52%). M.p. 220ºC.

IR (KBr) $cm^{-1}$: 3246, 3202, 3130, 2974, 2880, 1578, 1450, 1413, 1301, 1252, 1159, 1120, 947, 634, 335, 320.

$^1$H-NMR ($D_2O$): 1.2-1.4 (Sc, 3H), 2.3-2.6 (sc, 2H), 2.9 (s, 3H), 3.1-3.5 (sc, 4H).

Elemental analysis (%): (calculated) C 18.95 H 3.71 N 7.36; (found) C 19.37 H 3.78 N 6.99.

**EXAMPLE 2:**

Cis-dichloro(3-amino-1-ethyl-2-methylpyrrolidine)platinum$^{(II)}$ (I, R = Et, $R_1$ = Me)

In the same manner as in Example 1, this compound was obtained as a yellow solid (Yield 66%). M.p.

254-256ºC.
IR (KBr) $cm^{-1}$: 3211, 3136, 2976, 1595, 1446, 1392, 1175, 336, 315.
$^1$H-NMR ($D_2O$): 1.3 (t, J = 7Hz, 3H), 1.5 (sc, 3H), 1.9-2.6 (sc, 4H), 3.0-3.9 (sc, 4H).
Elemental analysis (%): (calculated) C 21.44 H 3.60 N 7.14; (found) C 21.24 H 3.86 N 7.03.

**EXAMPLE 3:**

Cis-dichloro(3-amino-2-ethyl-1-methylpyrrolidine)platinum$^{(II)}$ (I, R = Me, $R_1$ = Et)

In the same manner as in Example 1, this compound was obtained as a yellow solid (Yield 50%). M.p. 244-246ºC.
IR (KBr) $cm^{-1}$: 3246, 3204, 3130, 2974, 2880, 1593, 1450, 1415, 1304, 1253, 1174, 1159, 946, 634, 333, 318.
$^1$H-NMR ($D_2O$): 1.2-1.5 (sc, 5H), 2.3-2.6 (sc, 2H), 2.9 (s, 3H), 3.1-3.5 (sc, 4H).
Elemental analysis (%): (calculated) C 21.44 H 3.60 N 7.14; (found) C 21.40 H 3.97 N 6.81.

**EXAMPLE 4:**

Cis-dichloro(3-amino-1,2-diethylpyrrolidine)platinum$^{(II)}$ (I, R = $R_1$ = Et)

In the same manner as in Example 1, this compound was obtained as a yellow solid (Yield 70%). M.p. 209-210ºC.
IR (KBr) $cm^{-1}$: 3263, 3208, 3134, 2968, 2883, 1599, 1460, 1368, 1292, 1255, 1176, 1122, 958, 634, 325, 312.
$^1$H-NMR ($D_2O$): 1.0-1.3 (sc, 5H), 1.6 (t, J = 7Hz, 3H), 2.0-2.7 (sc, 2H), 3.2-3.8 (sc, 6H).
Elemental analysis (%): (calculated) C 23.66 H 3.97 N 6.89; (found) C 23.25 H 4.38 N 6.53.

**EXAMPLE 5:**

Cis-dichloro(3-amino-2-methyl-1-propylpyrrolidine)platinum$^{(II)}$ (I, R = Pr, $R_1$ = Me)

To a aqueous solution of 50 ml of ligand at 0.2M concentration 50 ml of a 0.2M $K_2PtCl_4$ aqueous solution were added. The system was kept continously under inert atmosphere. The mixture was stirred for 24 hours at room temperature. The solution was acidified to pH 1-2 by adding 2M HCl. The residue was concentrated to dryness and crystallized with 1M HCl to give a yellowish solid (Yield 30%). M.p. 226-229ºC-(d).
IR (KBr) $cm^{-1}$: 3235, 3204, 2980 2960, 2875, 1605, 335, 330.
Elemental analysis (%): (calculated) C 23.34 H 4.44 N 6.86; (found) C 23.53 H 4.21 N 6.75.

**EXAMPLE 6:**

Cis-dichloro(3-amino-1-hexyl-2-methylpyrrolidine)platinum$^{(II)}$ (I, R = Hexyl, $R_1$ = Me)

In the same manner as described in Example 5, this compound was obtained as a yellow solid (Yield 30%). M.p. 209-217ºC(d).
IR (KBr) $cm^{-1}$: 3230, 3208, 3140, 2980, 2950, 2880, 1610, 335, 330.
Elemental analysis (%): (calculated) C 29.34 H 5.37 N 6.22; (found) C 29.08 H 5.24 N 5.88.

**EXAMPLE 7:**

Cis-dichloro(3-amino-1-dodecyl-2-methylpyrrolidine)platinum$^{(II)}$ (I, R = Dodecyl, $R_1$ = Me)

In the same manner as described in Example 5, this compound was obtained as a yellow solid (Yield 31%). M.p. 194-197ºC(d).
IR (KBr) $cm^{-1}$): 3211, 3204, 2975, 2922, 2853, 1593, 1466, 1167, 326, 316.
$^1$H-NMR ($CDCl_3$): 0.9 (t, J = 7Hz, 3H), 1.1-1.3 (sc, 20H), 1.5-1.7 (sc, 5H), 2.1-3.1 (sc), 3.1.3.3 (sc), 3.3-4.1 (sc, 8H).

**EXAMPLE 8:**

Cis-dichloro(3-amino-1-ethyl-2-propylpyrrolidine)platinum(II) (I, R = Et, $R_1$ = Pr)

In the same manner as described in Example 5, this compound was obtained as a yellow solid (Yield 63%). M.p. 230-232ºC.
IR (KBr) $cm^{-1}$: 3238, 3206, 3142, 2961, 2895, 2866, 1589, 1468, 1367, 1296, 1248, 1159, 1130, 947,640, 335, 316.
$^1$H-NMR ($CDCl_3$): 0.95 (t, J = 7Hz, 3H), 1.0-1.2 (sc, 2H), 1.35-1.45 (sc, 2H), 1.49 (t, J = 7Hz, 3H), 2.0-2.3 (sc, 1H), 2.45-2.65 (sc, 1H), 2.95-3.2 (sc, 1H), 3.25-3.35 (sc, 1H), 3.45-3.55 (sc, 2H), 4.15-4,45 (sc, 2H).
Elemental analysis (%): (calculated) C 25.60 H 4.77 N 6.63 Cl 16.74; (found) C 25.83 H 4.70 N 6.60 Cl 16.88.

**EXAMPLE 9:**

Ethyl 5-methyl-4-nitro-1-propyl-2-pyrrolcarboxylate (VI, R = Pr, $R_1$ = Me)

2.4 g (0.01 mole) of ethyl 5-methyl-4-nitro-2-pyrrolcarboxylate potassium salt, 0.1 g (0.002 mole) of hexadecyltributylphosphonium bromide catalyzer and 50 ml of acetonitrile were placed into a flask. The mixture was heated on a thermostated bath at 80ºC under stirring. After 1.5 hours, the solid was filtered and the solvent removed under vaccum. 50 ml of ethyl ether were added. A spongy white solid precipitate (recovered catalyzer) was formed. Precipitation was enhanced by stirring. The solid was filtered through a 0.45 $\mu$m membrane and washed with ethyl ether. The filtered turbid liquid was taken through a $SiO_2$ column (10 g) and washed with ether. The solvent was removed by vaccum evaporation. 2.09 g (Yield 87%) of pale yellow oil were obtained.
IR (film) $cm^{-1}$: 3164, 2959, 1716, 1513, 1321, 751.
$^1$H-NMR ($CDCl_3$): 0.9 (t, J = 7Hz, 3H), 1.3 (t, J = 7Hz, 3H), 1.7 (sc, 2H), 2.6 (s, 3H), 4.3 (sc, 4H), 7.5 (s, 1H).
Elemental analysis (%): (calculated) C 54.99 H 6,71 N 11.66; (found) C 55.21 H 6.79 N 11.58.

**EXAMPLE 10:**

Ethyl 1-hexyl-5-methyl-4-nitro-2-pyrrolcarboxylate (VI, R = Hexyl, $R_1$ = Me)

2.4 (0.01 mole) of ethyl 5-methyl-4-nitro-2-pyrrolcarboxylate potassium salts, 0.1 g (0.002 mole) of hexadecyltributylphosponium bromide catalyzer and 50 ml of toluene were placed into a flask. The mixture was heated on a thermostated bath at 100ºC under stirring. 1,82 g (0.011 mole) of hexyl bromide was immediately added. At this point, the procedure described in Example 9 was used. 2.62 g (Yield 93%) of yellow oil were obtained
IR (film $CHCl_3$ evap.) $cm^{-1}$: 3151, 2964, 1716, 1512, 1321, 754.
$^1$H-NMR ($CDCl_3$): 0.9 (t, J = 7Hz, 3H), 1.3 (sc, 9H), 1.7 (sc, 2H), 2.6 (s, 3H9, 4.3 (Sc, 4H), 7.5 (s, 1H).
Elemental analysis (%): (calculated) C 59.55 H 7.85 N 9,92; (found) C 60.06 H 7.92 N 10.00.

**EXAMPLE 11:**

Ethyl 1-dodecyl-5-methyl-4-nitro-2-pyrrolcarboxylate (VI, R = Dodecyl, $R_1$ = Me)

2.4 g (0.01 mole) of ethyl 5-methyl-4-nitro-2-pyrrolcarboxylate potassium salt, 0.1 g (0.002 mole) of hexadecyltributylphosphonium bromide catalyzer and 50 ml of toluene were placed into a flask. The mixture was heated on a thermostated bath at 100ºC under stirring. 2.74 (0.011 mole) of dodecyl bromide were immediately added. At this point, the procedure described in Example 9 was used. 3.05 g (Yield 83%) of yellow oil were obtained.
IR (film) $cm^{-1}$: 3152, 2972, 1716, 1512, 1321, 754.
$^1$H-NMR ($CDCl_3$): 0.8 (t, J = 7Hz, 3H), 1.3 (sc, 21H), 1.7 (sc, 2H), 2.6 (s, 3H), 4.3 (sc, 4H), 7.5 (s, 1H).
Elemental analysis (%): (calculated) C 65.54 H 9.35 N 7.64; (found) C 65.56 H 9.45 N 7.63.

**EXAMPLE 12:**

1-Ethyl-4-nitro-propyl-2-pyrrolcarboxylic acid (VIII, R = Et, $R_1$ = Pr)

A solution of KOH was prepared in DMSO by stirring 7 g (0.1 mole) of 85% KOH in 20 ml of solvent under inert atmosphere. 2 g (0.01 mole) of ethyl 5-methyl-4-nitro-2-pyrrolcarboxylate were slowly added and stirred for 1 hour under a gentle dry nitrogen stream. Then, 15.6 g (0.1 mole) of ethyl iodide were added dropwise and the temperature of the mixture was kept within 30ºC. After stirring for 2 hours, the reaction mixture was poured onto 150 ml of water. The medium was verified to be alkaline by adding the necessary 2M KOH in order to attain pH >10. It was removed with ethyl ether. The aqueous phase was acidulated with 12M HCl. Precipitation of a whitish solid was observed. It was left to stand for 18 hours at 0ºC. It was filtered. The solid was dried on stove at 100ºC and recrystallized from toluene. 17 g (Yield 72%) of a white-brownish solid were obtained. M.p. 169-171ºC.
IR (KBr) $cm^{-1}$: 3500-2100, 3149, 2980, 1674, 1523, 1516, 752.
$^1H$-NMR ($CDCl_3$): 1.0 (t, J= 7Hz, 3H), 1.4 (t, J= 7Hz, 3H), 1.7 (sc, 2H), 3.0 (t, 2H), 4.4 (q, J= 7Hz, 3H), 7.6 (s, 1H).

**EXAMPLE 13:**

5-Methyl-4-nitro-1-propyl-2-pyrrolcarboxylic acid (VIII, R= Pr, $R_1$ = Me)

3.4 (0.014 mole) of ethyl 5-methyl-4-nitro-1-propyl-2-pyrrolcarboxylate, 80 ml of 2M NaOH and 30 ml of ethanol were placed into a 250-ml flask. The mixture was refluxed for 2 hours, concentrated to 30 ml approximately under reduced pressure and diluted with 250 ml of water. Then, it was filtered, cooled to 0ºC by adding ice and immediately acidulated with HCl. It was left to stand for 1 hour at 0ºC. A precipitate was observed. It was filtered and washed with water at 0ºC. The precipitate was treated with $NaHCO_3$ solution and filtered through a 0.45 µm membrane. The filtrate was acidulated with 6M HCl and left to stand for 18 hours at 0ºC. It was dried on phosphorus pentoxide under vaccum. 2.5 g (Yield 80%) of white-grayish solid were obtained. M.p. 136-138ºC.
IR (KBr) $cm^{-1}$: 3500-200, 3175, 2964, 1676, 1512, 1321, 752.
$^1H$-NMR ($CDCl_3$): 0.9 (t, J= 7Hz, 3H), 1.7 (sc, 2H), 2.7 (s, 3H), 4.3 (t, J= 7Hz, 2H), 7.6 (s, 1H), 11.4 (ps, 1H).

**EXAMPLE 14:**

1-Hexyl-5-methyl-4-nitro-2-pyrrolcarboxylic acid (VIII, R= Hexyl, $R_1$ = Me)

In the same manner as in Example 13, and from 3.5 g (0.012 mole) of ethyl 1-hexyl-5-methyl-4-nitro-2-pyrrolcarboxylate, 2.4 g (Yield 73%) of white-grayish solid are obtained. M.p. 167-169ºC.
IR (KBr) $cm^{-1}$: 3500-2100, 2953, 1691, 1520, 1321, 750.
$^1H$-NMR ($CDCl_3$): 0.9 (t, J= 7Hz, 3H), 1.0-1.4 (sc, 6H), 1.7 (sc, 2H), 2.7 (s, 3H), 4.3 (t, J= 7Hz, 2H), 7.6 (s, 1H).

**EXAMPLE 15:**

1-Dodecyl-5-methyl-4-nitro-2-pyrrolcarboxylic acid (VIII, R= Dodecyl, $R_1$ = Me)

In the same manner as described in Example 13, and from 4.5 g (o.o12 mole) of ethyl 1-dodecyl-5-methyl-4-nitro-2-pyrrolcarboxylate, 4.1 g (Yield 73%) of white-grayish solid are obtained. M.p. 83-86ºC.
IR (film $CH_2Cl_2$ evap.) $cm^{-1}$: 3500-2100, 3146, 1680, 1512, 1323, 750.
$^1H$-NMR ($CDI_3$): 0.8 (t, J= 7Hz, 3H), 1.2 (sc, 18H), 1.7 (sc, 2H), 2.7 (s, 3H), 4.3 (t, J= 7Hz, 2H), 7.6 (s, 1H).
Elemental analysis (%): (calculated) C 63.87 H 8.93 N 8.27; (found) C 63.83 H 8.76 N 8.29.

**EXAMPLE 16:**

2-Methyl-3-nitro-1-propylpyrrol (VII, R= Pr, $R_1$ = Me)

2.4 g (0.011 mole) of 5-methyl-4-nitro-1-propyl-2-pyrrolcarboxylic acid, 1 g of metallic copper powder and 10 ml of quinolein were placed into a 25-ml flask under a thermostatic bath at 195ºC for 1 hour. Then the mixture was cooled, diluted with 150 ml of diethyl ether and washed with 5x30 ml of 2M HCl and twice with sodium chloride saturated solution. It is dried over magnesium sulphate and the solvent was evaporated at reduced pressure to give a crude which, after purificationby column chromatography ($SiO_2$;

hexane:ether 5:1) affords 1.7 g (Yield 89%) yellow-orange oil.
IR (film) $cm^{-1}$: 3127, 2968, 1510, 1304, 723.
$^1H$-NMR ($CDCl_3$): 0.9 (t, J= 7Hz, 3H), 1.7 (sc, 2H), 2.6 (s, 3H), 3.8 (t, J= 7Hz, 2H), 6.4 (d, J= 3Hz, 1H), 6.7 (d, J= 3Hz, 1H).

**EXAMPLE 17:**

1-Hexyl-2-methyl-3-nitropyrrol (VII, R= Hexyl, $R_1$ = Me)

In the same manner as in Example 16, and from 2.4 (0.09 mole) of 1-hexyl-5-methyl-4-nitro-2-pyrrolcarboxilic acid, 1.7 g (Yield 89%) of yellow-reddish oil are obtained.
IR (film) $cm^{-1}$: 3125, 2967, 1512, 1304, 723.
$^1H$-NMR ($CDCl_3$): 0.8 (t, J= 7Hz, 3H, 1.3 (sc, 6H), 1.6 (sc, 2H), 2.6 (s, 3H), 3.8 (t, J= 7Hz, 2H), 6.4 (d, J= 3Hz, 1H), 6.7 (d, J= 3HZ, 1H).
Elemental analysis (%): (calculated) C 62.83 H 8.62 N 13.12; (found) C 62.68 H 8.45 N 13.75.

**EXAMPLE 18:**

1-Dodecyl-2-methyl-3-nitropyrrol (VII, R= Dodecyl, $R_1$ = Me)

In the same manner as in Example 16, and from 2.9 g (0.008 mole) of 1-dodecyl-5-methyl-4-nitro-2-pyrrolcarboxylic acid, 2.3 g (Yield 92%) of yellow-pale oil crystallizing below 20ºC are obtained.
IR (film) $cm^{-1}$: 3127, 2928,1508, 1305, 719.
$^1H$-NMR ($CDCl_3$): 0.8 (t, J= 7Hz, 3H), 1.2 (sc, 16H), 1.7 (sc, 2H), 2.6 (s, 3H), 3.8 (t, J= 7Hz, 2H), 6.4 (d, J= 3HZ, 1H), 6.7 (d, J= 3Hz, 1H).

**EXAMPLE 19:**

1-Ethyl-3-nitro-2-propylpyrrol (VII, R= Et, $R_1$ = Pr)

In the same manner as in Example 16, and from 2.3 (0.01 mole) of 1-ethyl-4-nitro-5-propyl-2-pyrrolcarboxylic acid, 1.58 g (Yield 87%) of yellowish oil crystallizing below 25ºC are obtained.
IR (film) $cm^{-1}$: 3146, 3127, 2290, 1514, 1302, 1172, 747.
$^1H$-NMR ($CDCl_3$): 1.0 (t, J= 7Hz, 3H, 1.5 (t, J= 7Hz, 3H), 1.6 (sc, 2H), 3.0 (t, J= 7Hz, 2H), 3.94 (q, J= 7Hz, 2H), 6.4 (d, J= 3HZ, 1H), 6.7 (d, J= 3Hz, 1H).

**EXAMPLE 20:**

3-Amino-2-methyl-1-propylpyrrolidine (II, R= Pr, $R_1$ = Me)

1.17 g (0.007 mole) of 2-methyl-3-nitro-1-propylpyrrol and 0.45 g of 5% $Rh/Al_2O_3$ catalyzer were placed into a hydrogenation reactor. 50 ml of isopropanol were slowly added and the reactor was sealed. It was cleansed with nitrogen and $H_2$ was introduced at 35 $kg/cm^2$ pressure. It was heated at 100ºC for 6 hours. The reactor was left to cool and then discharged. The turbid liquid was filtered through a 0.45 $\mu$m membrane under inert atmosphere. Isopropanol was removed by vaccum evaporation. The resulting liquid was distilled at reduced pressure (0.1-0.5 mmHg) to give 0.45 g (Yield 45%) of colourless, air-unstable oil.
Dipicrate (m.p. 214-215ºC):
IR (KBr) $cm^{-1}$: 3500-3200, 3080, 2970, 2935, 1570, 1495, 1545, 1330.
$^1H$-NMR ($CDCl_3$): 0.9 (t, J= 7Hz, 3H), 1.2-2.0 (sc, 7H), 2.0-2.4 (sc, 4H), 3.0-4.0 (sc, 6H), 8.6 (s, 4H).
Elemental analysis (%): (calculated) C 40.01 H 4.03 N 18.66; (found) C 39.97 H 3.92 N 18.25.

**EXAMPLE 21:**

3-Amino-1-hexyl-2-methylpyrrolidine (II, R= Hexyl, $R_1$ = Me)

In the same manner as in Example 20, and from 1.7 g (0.008 mole) of 1-hexyl-2-methyl-3-nitropyrrol and 0.35 g of $Rh/Al_2O_3$ 5% catalyzer, 0.9 g (Yield 60%) of yellow, air-unstable oil are obtained by distillation at reduced pressure (0.1-0.5 mmHg).

Dipricate:
IR (KBr) $cm^{-1}$: 3500-3200, 3080, 2960, 2930, 1569, 1493, 1546, 1332.
$^1$H-NMR ($CDCl_3$ + $d_6$-DMSO): 0.8 (t, J= 7Hz, 3H), 1.2-1.5 (sc, 8H), 1.6-1.8 (sc, 5H), 2.2-4.4 (sc, 10H), 8.6 (s, 4H).

**EXAMPLE 22:**

3-Amino-1-dodecyl-2-methylpyrrolidine (II, R= Dodecyl, $R_1$ = Me)

In the same manner as in Example 20, and from 1.9 g (0.0065 mole) of 1-dodecyl-2-methyl-3-nitropyrrol and 0.8 g of 5% $Rh/Al_2O_3$ catalyzer, 1.4 g (Yield 82%) of yellow, air-unstable oil are obtained by distillation at reduced pressure (0.1-0.5 mmHg).
Dipicrate (m.p. 169-171ºC).
IR (KBr) $cm^{-1}$: 3500-3200, 3080, 2960, 2930, 1570, 1405, 1545, 1330.
Elemental analysis (%): (calculated) C 47.93 H 5.82 N 15.41; (found) C 47.88 H 5.58 N 15.15.

**EXAMPLE 23:**

3-Amino-1-ethyl-2-propylpyrrolidine (II, R= Et, $R_1$ = Pr)

In the same manner as in Example 20, and from 2 g (0.011 mole) of 1-ethyl-3-nitro-2-propylpyrrol and 0.4 g of 5% $Rh/Al_2O_3$ catalyzer, 1.23 g (Yield 72%) of colourless oil are obtained by distillation at reduced pressure (3 mmHg).
Dipicrate (m.p. 166-170ºC).
IR (KBr) $cm^{-1}$: 3500-3200, 3080, 2966, 2934, 2874, 1452, 1149.
$^1$H-NMR ($d_6$-DMSO): 0.9 (t, J= 7Hzm 3H), 1.3 (d, J= 7Hz, 3H), 1.3-2.6 (sc, 6H), 3.0-3.9 (sc, 6H), 8.6 (s, 4H).

**Claims**

**1.** Aminopyrrolidine platinum compounds of the general formula (I):

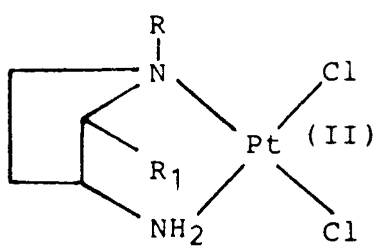

(I)

wherein R is a linear alkyl group containing 1 to 12 carbon atoms and $R_1$ is a linear alkyl group containing 1 to 3 carbon atoms.

**2.** A process for preparing the compounds according to Claim 1, characterized in that potassium tetrachloroplatinite is treated with the aminopyrrolidine of the general formula (II):

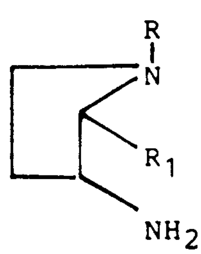

(II)

wherein R and $R_1$ are as defined for (I) in an aqueous medium.

3. Pharmaceutical compositions comprising at least one of the compounds of the Claim 1, optionally together with pharmaceutical carriers and/or adjuvants.

4. The use of the compounds according to Claim 1 for the preparation of pharmaceutical compositions for treating tumor diseases.

5. A process for preparing a pharmaceutical composition which comprises providing at least one compound of claim 1 in a form suitable for administration, optionally incorporated into pharmaceutical carriers and/or adjuvants.

European Patent Office

# EUROPEAN SEARCH REPORT

Application Number

EP 92 10 9480

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 327 709 (KANEBO LTD.)<br>* claims 1,10 *<br>--- | 1,3 | C07F15/00<br>A61K31/28 |
| A | EP-A-0 176 005 (CHUGAI SEIYAKU KABUSHIKI KAISHA)<br>* claims 1,33,34 *<br>----- | 1,3,4 | |
| | | | TECHNICAL FIELDS SEARCHED (Int. Cl.5) |
| | | | C07F<br>A61K |

The present search report has been drawn up for all claims

| Place of search | Date of completion of the search | Examiner |
|---|---|---|
| BERLIN | 03 SEPTEMBER 1992 | Hans Kapteyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document